(19) Europäisches Patentamt
European Patent Office
Office européen des brevets

(11) EP 1 326 845 B1

(12) **EUROPÄISCHE PATENTSCHRIFT**

(45) Veröffentlichungstag und Bekanntmachung des Hinweises auf die Patenterteilung:
**14.12.2005 Patentblatt 2005/50**

(21) Anmeldenummer: **01972078.8**

(22) Anmeldetag: **24.09.2001**

(51) Int Cl.[7]: **C07D 235/10**, A61K 31/4184, A61P 33/02

(86) Internationale Anmeldenummer:
**PCT/EP2001/011010**

(87) Internationale Veröffentlichungsnummer:
**WO 2002/030909 (18.04.2002 Gazette 2002/16)**

(54) **N-ALKOXYALKYL-SUBSTITUIERTE BENZIMIDAZOLE UND IHRE VERWENDUNG ALS MITTEL GEGEN PARASITÄRE PROTOZOEN**

N-ALKOXYALKYL-SUBSTITUTED BENZIMIDAZOLES AND THE USE THEREOF AS AN AGENT AGAINST PARASITIC PROTOZOANS

BENZIMIDAZOLES N-ALKOXYALKYL-SUBSTITUES ET LEUR UTILISATION EN TANT QU'AGENTS ANTI-PROTOZOAIRES PARASITES

(84) Benannte Vertragsstaaten:
**AT BE CH CY DE DK ES FI FR GB GR IE IT LI LU MC NL PT SE TR**

(30) Priorität: **06.10.2000 DE 10049468**

(43) Veröffentlichungstag der Anmeldung:
**16.07.2003 Patentblatt 2003/29**

(73) Patentinhaber: **Bayer HealthCare AG**
**51368 Leverkusen (DE)**

(72) Erfinder:
- **LIEB, Folker**
**51375 Leverkusen (DE)**
- **MARHOLD, Albrecht**
**51373 Leverkusen (DE)**
- **NEUGEBAUER, Torsten**
**53604 Bad Honnef (DE)**
- **GREIF, Gisela**
**53424 Remagen (DE)**

(56) Entgegenhaltungen:
**EP-A- 0 597 304** **EP-A- 0 602 465**
**WO-A-96/38140**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue N-Alkoxyalkyl-substituierte Benzimidazole, ihre Herstellung und ihre Verwendung als Mittel gegen parasitäre Protozoen.

Die vorliegende Erfindung betrifft ferner Mischungen dieser Verbindungen mit Polyetherantibiotika oder synthetisch hergestellten Coccidiosemitteln in Mitteln zur Bekämpfung parasitärer Protozoen, insbesondere Coccidien.

Substituierte Benzimidazole und ihre Verwendung als Insektizide, Fungizide und Herbizide sind bereits bekannt geworden (EP-OS 87 375, 152 360, 181 826, 239 508, 260 744, US-P 3 418 318, 3 472 865, 3 576 818).

Halogenierte Benzimidazole und ihre Wirkung als Anthelmintika, Coccidiostatika und Pestizide sind bekannt geworden (DE-OS 2 047 369, DE-OS 4 237 617). Mischungen von nitrosubsrituierten Benzimidazolen und Polyetherantibiotika sind als Coccidiosemittel bekannt geworden (US-P 5 331 003, US-P 5 670 485). Mischungen von anderen substituierten Benzimidazolverbindungen und Polyetherantibiotika oder synthetischen Coccidiosemitteln sind bekannt. (WO 96/38140, WO 00/04022).

Coccidiose ist eine Erkrankung, die durch einzellige Parasiten (Protozoen) hervorgerufen wird. Insbesondere bei der Geflügelaufzucht kann sie große Verluste hervorrufen. Um diese zu vermeiden, werden die Bestände prophylaktisch mit Coccidiosemitteln behandelt. Durch die Entwicklung von Resistenzen gegen die eingesetzten Mittel kommt es schon kurz nach Einführung der Mittel zu ernsthaften Problemen. Durch den Einsatz chemisch völlig neuer Coccidiosemittel, insbesondere Kombinationen, ist es andererseits möglich, auch polyresistente Parasitenstämme zu kontrollieren.

Es besteht daher nach wie vor ein Bedarf an neuen Wirkstoffen und Mitteln mit verbesserten Eigenschaften bei der Bekämpfung von Krankheiten, die durch parasitäre Protozoen hervorgerufen werden.

Die Erfindung betrifft neue Benzimidazole der Formel (I)

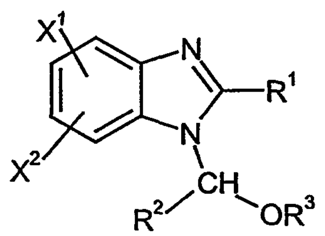

(I),

in welcher

$R^1$ für Fluoralkyl steht,

$R^2$ für Wasserstoff oder Alkyl steht,

$R^3$ für Alkyl steht,

$X^1$ für Trifluormethyl steht

$X^2$ für Trifluormethoxy steht,

gefunden, die eine hervorragende Wirksamkeit gegen Coccidiose zeigen.

Die Benzimidazole der Formel (I)

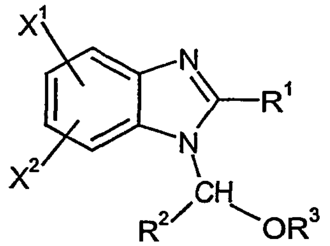

(I),

in welcher
$R^1$, $R^2$, $R^3$, $X^1$ und $X^2$ die obengenannten Bedeutungen haben,
werden hergestellt, indem man
1 H-Benzimidazole der Formel (II)

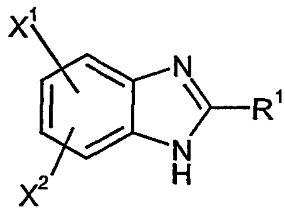

(II),

in welcher
R1, $X^1$ und $X^2$ die oben angegebene Bedeutung haben,
mit einem Alkylierungsmittel der Formel (III)

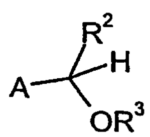

(III),

in welcher

A für eine geeignete Abgangsgruppe steht,

$R^2$ und $R^3$ die oben angegebene Bedeutung haben,

gegebenenfalls in Gegenwart von Verdünnungsmitteln und/oder Reaktionshilfsmitteln umsetzt.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Die Isomere können gegebenenfalls in an sich bekannter Weise getrennt werden, z.B. durch Kristallisations- oder Chromatographieverfahren. Sowohl die reinen Isomeren als auch die Isomerengemische werden erfindungsgemäß beansprucht.

Die erfindungsgemäßen Verbindungen können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon-oder Sulfonsäuren wie beispielsweise Essigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Die erfindungsgemäßen substituierten Benzimidazole sind durch die Formel (I) allgemein definiert. Bevorzugt sind Verbindungen der Formel (I), bei welchen

$R^1$ für $C_1$-$C_4$-Fluoralkyl steht,

$R^2$ für Wasserstoff oder $C_1$-$C_4$-Alkyl steht,

$R^3$ für $C_1$-$C_8$-Alkyl steht,

$X^1$ für Trifluormethyl steht

$X^2$ für Trifluormethoxy steht,

Besonders bevorzugt sind Verbindungen der Formel (I), in welcher

$R^1$ für $CF_3$, $CHF_2$, CHF steht,

$R^2$ für Wasserstoff, Methyl, Ethyl, n-Propyl oder Isopropyl steht,

$R^3$ für $C_1$-$C_6$-Alkyl steht,

$X^1$ für Trifluormethyl steht

$X^2$ für Trifluormethoxy steht,

Ganz besonders bevorzugt sind Verbindungen der Formel (I), in welcher

$R^1$ für $-CF_3$ steht

$R^2$ für Wasserstoff steht,

$R^3$ für Methyl, Ethyl, Propyl oder iso-Propyl, Butyl, iso-Butyl oder sec.- Butyl steht,

$X^1$ für Trifluormethyl steht

$X^2$ für Trifluormethoxy steht,

Alkyl steht im Allgemeinen für einen geradkettigen oder verzweigten Alkylrest mit bevorzugt bis zu 8, besonders bevorzugt bis zu 6, ganz besonders bevorzugt bis zu 4 Kohlenstoffatomen. Beispiele sind: Methyl, Ethyl, n-Propyl, iso-Propyl, n-Butyl, iso-Butyl (= sec.-Butyl).

Fluoralkyl steht im Allgemeinen für einen geradkettigen oder verzweigten Alkylrest mit bevorzugt bis zu 6, besonders bevorzugt bis zu 4, ganz besonders bevorzugt bis zu 3 Kohlenstoffatomen, in dem mindestens ein bevorzugt mehrere, besonders bevorzugt alle Wasserstoffe durch Fluor ersetzt sind. Beispiele sind: Trifluormethyl, Difluormethyl, Pentafluorethyl etc.

Verwendet man zur Durchführung des erfindungsgemäßen Verfahrens zur Herstellung von Verbindungen der Formel (I) beispielsweise 5-Trifluormethoxy-2,6-bis-trifluormethylbenzimidazol, so lässt sich der Reaktionsablauf des Herstellungsverfahrens durch das folgende Formelschema darstellen:

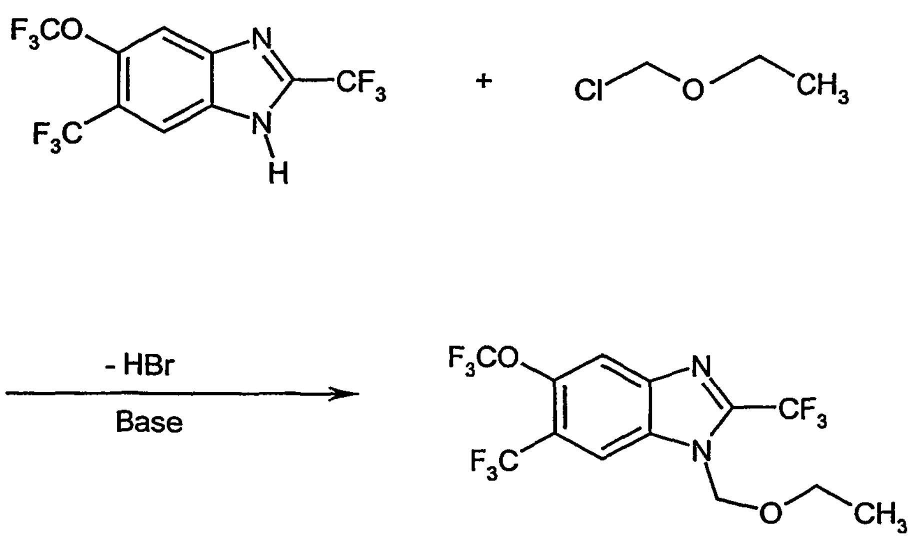

Die zur Durchführung des Herstellungsverfahrens als Ausgangsstoffe benötigten 1H-Benzimidazole sind durch die Formel (II) allgemein definiert. In dieser Formel (II) stehen $R^1$ bis $R^3$ sowie $X^1$ und $X^2$ vorzugsweise für diejenigen Reste, die bereits im Zusammenhang mit der Beschreibung der erfindungsgemäßen Verbindungen der Formel (I) als bevorzugt für diese Substituenten genannt wurden.

Die 1H-Benzimidazole der Formel (II) sind bekannt oder erhältlich in Analogie zu bekannten Verfahren (vergl. z.B. J. Amer. Chem. Soc. 75, 1292 [1953], US-Patent 3.576.818)

Die zur Durchführung des Herstellungsverfahrens weiterhin als Ausgangsprodukte erforderlichen Alkylierungsmittel sind durch die Formel (III) allgemein definiert. In dieser Formel (III) stehen $R^2$ und $R^3$ vorzugsweise für diejenigen Reste, die bereits im Zusammenhang mit der Beschreibung der erfindungsgemäßen Stoffe der Formel (I) als bevorzugt für diesen Substituenten genannt wurden.

A steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

Die Verbindungen der Formel (III) sind allgemein bekannt oder erhältlich in Analogie zu bekannten Verfahren (vergl. z.B J. Amer. Chem. Soc. 93, 5725-5731, Synthesis 1982, 942-944)

Als Verdünnungsmittel zur Durchführung des Herstellungsverfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Düsopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester, oder Basen wie Pyridin.

Das Herstellungsverfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Lithium-diethylamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, Lithium-organische Verbindungen, wie n-Butyllithium sowie tertiäre Amine, wie Trimethylamin, Triethylamin, Tributylamin, Di-isopropyl-ethylamin, Tetramethylguanidin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Das Herstellungverfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}/C_{15}$-alkylammoniumchlorid, Trimethyl-$C_{13}/C_{15}$-alkylammoniumbromid, Dibenzyl-dimethyl-ammoniummethylsulfat, Dimethyl-$C_{12}/C_{14}$-alkylbenzylammoniumchlorid, Dimethyl-$C_{12}/C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl)-amin.

Die Reaktionstemperaturen können bei der Durchführung des Herstellungsverfahrens in einem größeren Bereich variiert werden. Im allgemeinen arbeitet man bei Temperaturen zwischen -70°C und +200°C, vorzugsweise bei Temperaturen zwischen 0°C und 130°C.

Das Herstellungsverfahren wird üblicherweise unter Normaldruck durchgeführt. Es ist jedoch auch möglich unter erhöhtem oder vermindertem Druck zu arbeiten.

Zur Durchführung des Herstellungsverfahrens setzt man pro Mol an 1H-Benzimidazol der Formel (II) im allgemeinen 1,0 bis 5,0 Mol, vorzugsweise 1,0 bis 2,5 Mol an Alkylierungsmittel der Formel (III) und gegebenenfalls 0,01 bis 5,0 Mol, vorzugsweise 1,0 bis 3,0 Mol an Reaktionshilfsmittel ein.

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen - insbesondere bei Regioisomerengemischen - mit Hilfe der Protonen-Kernresonanzspektroskopie ($^1$H-NMR).

Die Wirkstoffe eignen sich bei günstiger Warmblütertoxizität zur Bekämpfung von parasitischen Protozoen, die in der Tierhaltung und Tierzucht bei Nutz-, Zucht-, Zoo-, Labor-, Versuchs- und Hobbytieren vorkommen. Sie sind

dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana,E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, L canis, L felis, L ohioensis, I. rivolta, I. spec., I. suis, Neospora caninum, Neospora hughesi; Cystisospora spec., Cryptosporidium spec. wie Toxoplasmadidae z.B. Toxoplasma gondii, wie Sarcocystidae z.B. Sarcocystis bovicanis, S. bovihominis, S. neurona, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz-und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut, z.B. Karpfen von 2 bis 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und desEinpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgussformulierungen, Gele;

Emulsionen und Suspension zur oralen oder dermalen Anwendung sowie zur Injektion; Halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden, wie oben bei den Injektionslösungen beschrieben, hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die aufgetragen oder aufgestrichen oder in Körperhöhlen eingebracht. Gele werden hergestellt, indem Lösungen, die wie bei den Injektionslösungen beschrieben hergestellt worden sind, mit soviel Verdikkungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole,Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder von Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichenFettsäuren, Partialglyceridgemische gesättigter oder ungesättigter, eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, iso-

propylstearat, Ölsäureoleylester,Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz; kationaktive Tenside wie Cetyltrimethylammoniumchlorid.

Als weitere Hilfsstoffe seien genannt:
Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Besonders hervorgehoben seien Mischungen der erfindungsgemäßen Verbindungen mit einem Polyetherantibiotikum oder einem synthetisch hergestellten Coccidiosemittel.

Als synthetische Coccidiosemittel bzw. als Polyetherantibiotika zur Verwendung in den erfindungsgemäßen Mischungen seien bevorzugt genannt:

Amprolium, z.T. in Kombination mit Folsäureantagonisten
Robenidin
Toltrazuril
Monensin
Salinomycin
Maduramicin
Lasalocid
Narasin
Semduramicin.

Aus dieser Liste bevorzugt sind Monensin, Salinomycin und Maduramicin. Besonders hervorgehoben sei die Mischung mit Maduramicin.

Anwendungsfertige Zubereitungen enthalten die Wirkstoffe in Konzentrationen von 10 ppm bis 20 Gewichtsprozent, bevorzugt von 0,1 bis 10 Gewichtsprozent.

Zubereitungen die vor Anwendung verdünnt werden, enthalten den Wirkstoff in Konzentrationen von 0,5 bis 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

In der Mischung mit anderen Coccidiosemitteln oder Polyetherantibiotika liegen die erfindungsgemäßen Wirkstoffe im Verhältnis 1 zu 0,1 - 10 bis 1 zu 1 - 10 vor. Bevorzugt ist das Verhältnis 1 zu 5.

Die Wirkstoffe können auch zusammen mit dem Futter oder Trinkwasser der Tiere verabreicht werden.

Futter- und Nahrungsmittel enthalten 0,01 bis 250 ppm, vorzugsweise 0,5 bis 100 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten.

Die hierbei erhaltene Vormischung kann dann dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muss. Bei der Verabreichung größerer Mengen kann es zweckmäßig sein, diese im Verlauf des Tages in mehrere Einzeldarreichungen zu unterteilen.

Die Wirksamkeit der erfindungsgemäßen Verbindungen lässt sich z.B. in Käfigversuchen mit folgender Versuchsanordnung belegen, bei der die Tiere mit den jeweiligen Einzelkomponenten sowie mit den Mischungen der Einzelkomponenten behandelt werden.

Ein wirkstoffhaltiges Futter wird so zubereitet, dass die erforderliche Menge Wirkstoff mit einem nährstoffmäßig ausgeglichenen Tierfutter, z.B. mit dem unter angegebenen Kükenfutter, gründlich vermischt wird.

Wenn ein Konzentrat oder eine Vormischung zubereitet werden soll, die schließlich im Futter auf die im Versuch genannten Werte verdünnt werden soll, werden im allgemeinen etwa 1 bis 30 %, vorzugsweise etwa 10 bis 20 Gew.-% Wirkstoff mit einem eßbaren organischen oder anorganischen Träger, z.B. Mais- und Sojamehl oder Mineralsalzen, die eine kleine Menge eines eßbaren Entstäbungsöls, z.B. Maisöl oder Sojabohnenöl enthalten, vermischt. Die so erhaltene Vormischung kann dann dem vollständigen Geflügelfutter vor der Verabreichung zugegeben werden.

Als Beispiel für die Verwendung der erfindungsgemäßen Stoffe im Geflügelfutter kommt die folgende Zusammensetzung in Frage.

| | |
|---|---|
| 52,00 % | Futtergetreideschrot, und zwar: 40 % Mais, 12 % Weizen |
| 17,00 % | Sojaschrot extr. |
| 5,00 % | Maisklebefutter |
| 5,00 % | Weizenfuttermehl |
| 3,00 % | Fischmehl |
| 3,00 % | Mineralstoffmischung |
| 3,00 % | Luzernegrasgrünmehl |
| 2,50 % | Vitaminvormischung |
| 2,00 % | Weizenkeime, zerkleinert |
| 2,00 % | Sojaöl |

(fortgesetzt)

| | |
|---|---|
| 2,00 % | Fleischknochenmehl |
| 1,50 % | Molkenpulver |
| 1,00 % | Melasse |
| 1,00 % | Bierhefe, gebunden an Biertreber |
| 100,00 % | |

Ein solches Futter enthält 18 % Rohprotein, 5 % Rohfaser, 1 % Ca, 0,7 % P sowie je kg 1200 i.E. Vitamin A, 1200 i.E. Vitamin D3, 10 mg Vitamin E, 20 mg Zinkbacitracin.

Diesem Futter wird der Wirkstoff in Mengen von z.B. 1 bis 20 ppm (w/w) zugemischt. Geeignete Dosierungen des Wirkstoffs sind z.B. 1 ppm; 2,5 ppm; 5 ppm (jeweils angegeben als Gewichtsanteile "(w/w)").

## Herstellungsbeispiele Verbindungen der Formel (I)

### Beispiel 1

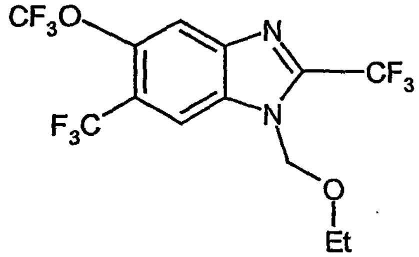

0,85 g (2,5 mmol) 5-Trifluormethoxy 2,6-bis-trifluormethyl-benzimidazol legt man in 25 ml abs. Methylenchlorid vor, gibt 0,45 ml (0,32 g) (3,15 mmol) Triethylamin bei 20°C zu. Anschließend tropft man bei 20°C 0,30 g (3,15 mmol) Chlormethyl-ethylether in 2,5 ml abs Methylenchlorid zu und refluxiert 24 h. Man wäscht die organische Phase dreimal mit Wasser, mit wässriger Natriumchloridlösung, trocknet über Natriumsulfat und dampft ein. Der Rückstand wird an Kieselgel (35-70 µm) mit Cyclohexan/Ethylacetat (5:1) chromatographiert.

Ausb.: 0,54 g (54 % d. Theorie), Öl. $R_F$= 0,47 [DC: Cyclohexan/Ethylacetat (5:1)], Regioisomerengemisch (Verhältnis 1: 1).

### Beispiel 2

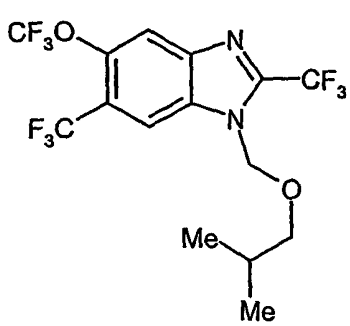

1,7 g (5 mmol) 5-Trifluormethoxy 2,6-bis-trifluormethyl-benzimidazol legt man in 50 ml abs. Methylenchlorid vor, gibt 0,9 ml (0,64 g) (6,3 mmol) Triethylamin bei 20°C zu. Anschließend tropft man bei 20°C 0,65 g (6,3 mmol) Chlormethyl-2-methyl-i-propylether in 5 ml abs Methylenchlorid zu und refluxiert 16 h. Man wäscht die organische Phase zweimal mit Wasser, mit wässriger Natriumchloridlösung, trocknet über Natriumsulfat und dampft ein. Der Rückstand wird an Kieselgel (35-70 µm) mit Cyclohexan/Ethylacetat (10:1) chromatographiert.
Ausb.: 1,0 g (47 % d. Theorie), Öl. $R_F$= 0,48 [DC: Cyclohexan/Ethylacetat (5:1)], Regioisomerengemisch (Verhältnis 1:1).

<u>**Beispiel 3**</u>

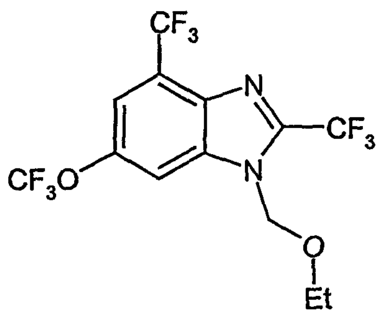

0,68 g (2 mmol) 6-Trifluormethoxy 2,4-bis-trifluormethyl-benzimidazol legt man in 20 ml Methylenchlorid vor, gibt 0,35 ml (0,25 g) (2,5 mmol) Triethylamin bei 20°C zu. Anschließend tropft man bei 20°C, 0,19 g (2, 5 mmol) Chlormethyl-ethylether in 2,5 ml abs Methylenchlorid zu und refluxiert 16 h. Man wäscht die organische Phase dreimal mit Wasser, mit wässriger Natriumchloridlösung, trocknet über Natriumsulfat und dampft ein. Der Rückstand wird an Kieselgel (35-70 µm) mit Cyclohexan/Ethylacetat (5:1) chromatographiert.
Ausb.: 0,63 g (76 % d. Theorie), Öl. $R_F$= 0,5 [DC: Cyclohexan/Ethylacetat (5:1)].

**Herstellung der Ausgangsverbindungen für die Beispiele 1 und 2**

<u>**Beispiel a**</u>

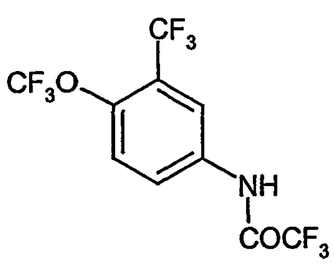

122 g (0,5 mol) 4-Trifluormethoxy-3-trifluormethylanilin legt man in 500 ml Toluol und 5 ml Pyridin bei Raumtemperatur vor, tropft 112 g (1,1 mol) Trifluoressigsäure zu und erhitzt 3 h unter Rückfluss am Wasserabscheider. Man dampft ein, versetzt den Rückstand mit Wasser, extrahiert mit Methylenchlorid und trocknet die organische Phase mit Natriumsulfat. Die organische Phase wird eingedampft und im Vakuum destilliert.
Ausb.: 109 g (64 % d. Theorie), $Kp_{0,1}$: 120-125°C, GC: 95,8 %.

**Beispiel b**

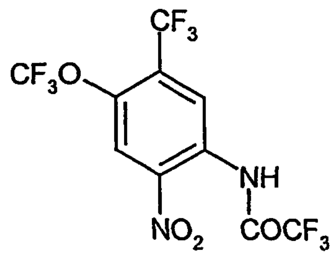

110 g (0,32 mol) N-(4-Trifluormethoxy-3-trifluormethyl-phenyl)-tifluoracetamid werden in 180 ml konz. Schwefelsäure unter Kühlung gelöst. Man tropft unter Eiskühlung bei 5°C eine Mischung aus 180 ml konz. Schwefelsäure und 30 ml konz. Salpetersäure langsam in 5 h zu. Dann gießt man den Ansatz auf Eis, extrahiert mit Methylenchlorid, wäscht die organische Phase mit Wasser neutral und trocknet über Natriumsulfat. Die organische Phase wird eingedampft und im Vakuum getrocknet. Das Rohprodukt wird weiter umgesetzt.
Ausb.: 95 g (77 % d. Theorie), GC: > 99 %.

**Beispiel c**

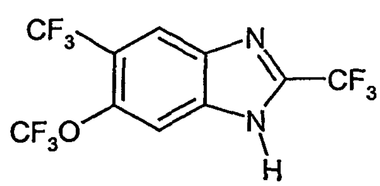

35 g (0,09 mol) N-(2-Nitro-4-trifluormethoxy-5-trifluormethyl-phenyl)-trifluoracetamid werden im Autoklaven mit 8 g Raney-Nickel in 200 ml Methanol bei 100 bar Wasserstoffdruck 22 h hydriert. Anschließend wird das Raney-Nickel abfiltriert, mit Methanol gewaschen und die Mutterlauge eingedampft. Der Rückstand wird mit Methylenchlorid versetzt, mit wenig Wasser zweimal gewaschen und über Natriumsulfat getrocknet. Der Rückstand enthält noch N-(2-Amino-4-trifluormethoxy-5-trifluormethyl-phenyl)-trifluoracetamid (GC). Deshalb wird die organische Phase mit 150 ml Trifluoressigsäure und 20 ml Trifluoressigsäureanhydrid versetzt und 14 h unter Rückfluss erhitzt. Der Ansatz wird abgekühlt und mit 200 ml 20%ige Natriumhydroxidlösung und nochmals mit 50 ml konz. Natriumhydroxid auf pH 8 gestellt. Man extrahiert mit Methylenchlorid, wäscht die organische Phase mit Wasser neutral, trocknet über Natriumsulfat und dampft die organische Phase ein.
Ausb.: 10 g (32 % d. Theorie), GC: 64 %.

**Herstellung der Ausgangsverbindung für das Beispiel 3**

**Beispiel d**

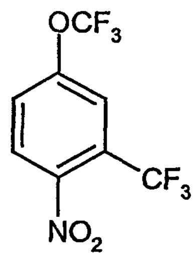

115 g (0,5 mol) 3-Trifluormethylbenzotrifluorid werden in 180 ml konz. Schwefelsäure unter Kühlung gelöst. Man tropft unter Eiskühlung bei 5°C eine Mischung aus 180 ml konz. Schwefelsäure und 30 ml konz. Salpetersäure langsam in 5 h zu, gießt den Ansatz dann auf Eis, extrahiert mit t-Butyl-methylether, wäscht die organische Phase mit Wasser neutral und trocknet über Natriumsulfat. Die organische Phase wird eingedampft und im Vakuum getrocknet.
Ausb.: 90 g (65 % d. Theorie), GC: > 98 %.

**Beispiel e**

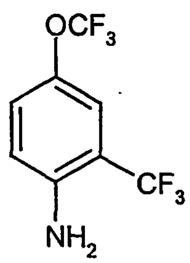

30 g (0,19 mol) 4-Trifluormethoxy-2-trifluormethyl-nitrobenzol werden im Autoklaven mit 2 g Raney-Nickel in 200 ml Methanol bei 100 bar Wasserstoffdruck hydriert. Anschließend wird das Raney-Nickel abfiltriert, mit Methanol gewaschen und die organischen Phasen eingedampft. Der Rückstand wird mit Methylenchlorid versetzt, mit wenig Wasser zweimal gewaschen und über Natriumsulfat getrocknet.
Ausb.: 23,7 g (89 % d. Theorie), GC:> 96 %.

**Beispiel f**

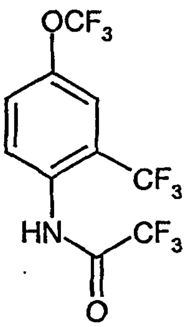

23,7 g (0,97 mol) 4-Trifluormethoxy-2-trifluormethylanilin legt man in 400 ml Methylenchlorid vor, gibt 58,6 g (0,58 mol) Triethylamin zu. Anschließend tropft man bei 20°C 61 g (0,29 mol) Trifluoressigsäureanhydrid zu und rührt 1 h bei RT nach. Man wäscht die organische Phase mit Wasser, trocknet über Natriumsulfat und dampft ein. Man destilliert den Rückstand im Vakuum.
Ausb.: 17,8 g (54 % d. Theorie), $Kp_{0,5}$: 68°C, GC: 93,7 %.

**Beispiele g**

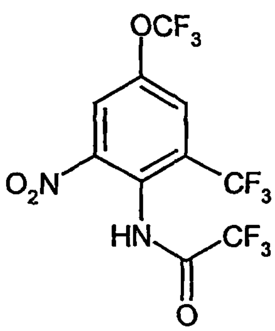

18 g (0,53 mol) N-(4-Trifluormethoxy-2-trifluormethyl)-trifluoracetamid werden in 50 ml konz. Schwefelsäure unter Kühlung gelöst. Man tropft unter Eiskühlung bei 5°C eine Mischung aus 50 ml.konz. Schwefelsäure und 10 ml konz. Salpetersäure langsam in 2 h zu. Dann gießt man den Ansatz auf Eis, extrahiert mit t-Butyl-methylether, wäscht die organische Phase mit Wasser und Natriumchloridlösung neutral, trocknet über Natriumsulfat. Die organische Phase wird eingedampft und im Vakuum getrocknet. Umkritallisation aus Ethanol. Ausb.: 13,2 g (65 % d. Theorie) GC: 96 %.

**Beispiel h**

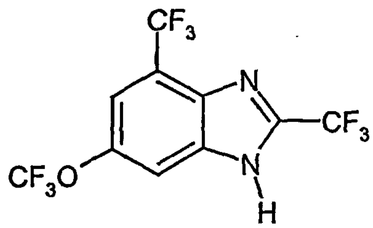

5 g (12,9 mmol) N-(2-Nitro-4-trifluormethoxy-6-trifluormethyl)-trifluoracetamid werden im Autoklaven mit 1 g Raney-Nikkel in 50 ml Methanol bei 100 bar Wasserstoffdruck 8 h hydriert. Anschließend wird das Raney-Nickel abfiltriert, mit Methanol gewaschen und die organische Phase eingedampft. Der Rückstand wird mit Methylenchlorid versetzt, mit wenig Wasser zweimal gewaschen und über Natriumsulfat getrocknet. Der Rückstand enthält noch N-(2-Amino-4-trifluormethoxy-6-trifluormethyl)-trifluoracetamid (GC). Daher wird der die organische Phase mit 25 ml Trifluoressigsäure und 5 ml Trifluoressigsäureanhydrid versetzt und 5 h unter Rückfluss erhitzt. Der Ansatz wird abgekühlt und mit 20 %ige Natriumhydroxidlösung auf pH 8 eingestellt. Man extrahiert mit Methylenchlorid, wäscht die organische Phase mit Wasser neutral, trocknet über Natriumsulfat und dampft ein. Der Rückstand wird in Vakuum getrocknet.
Ausb.: 4,1 g (94 % d, Theorie), GC: 85,6 %.

**Biologische Beispiele**

**Käfigversuch Coccidiose/Küken**

Coccidienfrei aufgezogene 8 bis 12 Tage alte männliche Hühnerküken (z.B. LSL Brinkschulte/Senden) erhalten von 3 Tage vor (Tag -3) der Infektion (= a.i.) bis 8 (9) Tage nach der Infektion (= p.i.) die erfindungsgemäßen Verbindungen (Testsubstanzen) in der in ppm angegebenen Konzentration mit dem Futter. In jedem Käfig werden 3 Tiere gehalten. Je Dosierung werden ein bis mehrere derartige Gruppen eingesetzt. Die Infektion erfolgt mittels einer Schlundsonde direkt in den Kropf mit etwa 100.000 sporulierten Oocysten von Eimeria acervulina sowie mit jeweils etwa 30.000 Oocysten von E. maxima und 40.000 sporulierten Oocysten von E. tenella. Es handelt sich hierbei um hochvirulente Stämme. Die genaue Infektionsdosis wird so eingestellt, dass möglichst eins von drei experimentell infizierten unbehandelten Küken infektionsbedingt stirbt. Für die Beurteilung der Wirksamkeit werden die folgenden Kriterien berücksichtigt: Gewichtszunahme von Versuchsbeginn bis Versuchsende, infektionsbedingte Sterberate, makroskopische Beurteilung der Faeces hinsichtlich Durchfall und Blutausscheidung an den Tagen 5 und 7 p.i. (Bewertung 0 bis 6), makroskopische Beurteilung der Darmschleimhaut, insbesondere der Blinddärme (Bewertung 0 bis 6) und die Oocystenausscheidung sowie der Anteil (in %) der innerhalb von 24 Stunden sporulierenden Oocysten. Die Zahl der Oocysten im Kot wurde mit Hilfe der McMaster-Zählkammer bestimmt (siehe Engelbrecht und Mitarbeiter "Parasitologische Arbeitsmethoden in Medizin und Veterinärmedizin, Akademie-Verlag, Berlin (1965)). Die einzelnen Befunde werden in Relation zu den unbehandelten nicht infizierten Kontroll-Gruppen gesetzt und eine Gesamtbewertung errechnet (vgl. A. Haberkorn (1986), S. 263 bis 270 in Research in Avian Coccidiosis ed L.R McDougald, L.P. Joyner, P.L. Long Proceedings of the Georgia Coccidiosis Conference Nov, 18.-20.1985 Athens/Georgia USA).
Versuchsergebnisse mit erfindungsgemäßen Kombinationen sind in den folgenden Tabellen beispielhaft aufgeführt. Die synergistische Wirksamkeit der Kombinationen im Vergleich zu den Einzelkomponenten wird besonders an der Reduktion der Oocystenausscheidung aber auch bezüglich der Sektionsbefunde, Gewichtsentwicklung und besseren Verträglichkeit ersichtlich.
In den folgenden Tabellen bedeutet in Spalte "Treatment" die Angabe

| | |
|---|---|
| n.inf.contr. = | nicht infizierte Kontrollgruppe |
| inf.contr. = | infizierte Kontrollgruppe |
| 1 = | Benzimidazol Beispiel Nr. |

In der Spalte "ppm" wird die eingesetzte Konzentration des Wirkstoffs im Futter in ppm angegeben.
In der Spalte "mortality" wird angegeben unter % der Prozentsatz der gestorbenen Tiere und unter n die Anzahl der gestorbenen Tiere/im Versuch eingesetzten Tiere.

In der Spalte "weight % of not inf. control" wird das Verhältnis des Gewichts der behandelten Tiere zum Gewicht der nicht infizierten Kontrollgruppe angegeben.

In den Spalten "dropping scores", "lesion score" und "oocyst control" werden Einzelangaben zur Wirkung gemacht.

In der Spalte "% efficacy" wird die Gesamtwertung bonitiert; 0 % bedeutet keine Wirkung, 100 % bedeutet volle Wirkung.

In den folgenden Tabellen werden die Ergebnisse der Wirksamkeitsversuche mit den erfindungsgemäßen Verbindungen zusammengefasst:

**Tabelle 1**

**Wirksamkeit gegen Eimeria acervulina, Eimeria maxima und Eimeria tenella**

| Verb. | 50 ppm | 25 ppm | 10 ppm | 7,5 ppm | 5 ppm | 2,5 ppm | 1 ppm |
|---|---|---|---|---|---|---|---|
| Bsp. 1 | N T | N T | 2 2 1 | 2 2 2 | 2 2 2 | 2 2 2 | 0 1 1 |
| Bsp. 2 | 2 2 2 | 2 2 2 | 1 2 1 | 1 1 1 | 0 1 0 | 1 1 1 | 1 1 1 |
| Bsp. 3 | N T | N T | 1 1 1 | 2 2 2 | 2 2 2 | 2 2 1 | 0 0 0 |

**Bewertungsschema**

2 = volle Wirkung
1 = schwache Wirkung
0 = unwirksam
T = Tod
N T= nicht getestet

Tabelle 2

Experimentelle Infektion mit Eimeria acervulina, Eimeria maxima und Eimeria tenella an Küken. Bsp. 2 in Kombination mit Maduramicin (MAD)

| Treatment | ppm | mortality | | weight % of not inf. control | dropping scores | lesion score | oocyst in % of inf. control | | | | % efficay tot. |
|---|---|---|---|---|---|---|---|---|---|---|---|
| | | % | n | | | | ac. | max. | ten. | tot. | |
| n. inf. contr. | 0 | 0 | 0/6 | 100 | 0 | 0 | 0 | 0 | 0 | 0 | 100 |
| inf. contr. | 0 | 0 | 0/6 | 46 | 6 | 6 | 100 | 100 | 100 | 100 | 0 |

Tabelle 2 (fortgesetzt)

| Experimentelle Infektion mit Eimeria acervulina, Eimeria maxima und Eimeria tenella an Küken. Bsp. 2 in Kombination mit Maduramicin (MAD) | | | | | | | | | | | |
|---|---|---|---|---|---|---|---|---|---|---|---|
| Treatment | ppm | mortality | | weight % of not inf. control | dropping scores | lesion score | oocyst in % of inf. control | | | | % efficay tot. |
| | | % | n | | | | ac. | max. | ten. | tot. | |
| MAD | 1 | 67 | 2/3 | 17 | 6 | 6 | >100 | >100 | 85 | >100 | 0 |
| MAD | 2 | 0 | 0/3 | 82 | 2 | 6 | >100 | >100 | >100 | >100 | 29 |
| MAD | 3 | 0 | 0/3 | 98 | 0 | 1 | >100 | >100 | 75 | >100 | 44 |
| Bsp.2 | 2,5 | 33 | 1/3 | 33 | 6 | 6 | >100 | >100 | >100 | >100 | 2 |
| Bsp. 2 | 5 | 33 | 1/3 | 65 | 6 | 6 | 54 | 0 | 98 | 73 | 37 |
| Bsp. 2 | 7,5 | 33 | 1/3 | 74 | 6 | 6 | 56 | 0 | 23 | 41 | 36 |
| Bsp. 2 | 10 | 0 | 0/3 | 69 | 6 | 6 | 93 | >100 | 65 | 83 | 17 |
| Bsp. 2 + MAD | 10 + 1 | 0 | 0/3 | 118 | 0 | 1 | 0 | 0 | 0 | 0 | 98 |
| Bsp. 2 + MAD | 7,5 2 | 0 | 0/3 | 103 | 0 | 1 | 4 | 0 | 1 | 3 | 93 |
| Bsp. 2 + MAD | 10 + 2 | 0 | 0/3 | 113 | 0 | 0 | 0 | 0 | 0 | 0 | 100 |
| Bsp. 2 + MAD | 2,5 + 3 | 0 | 0/3 | 115 | 0 | 2 | 1 | 30 | 3 | 2 | 78 |
| Bsp.2 + MAD | 5 + 3 | 0 | 0/3 | 135 | 0 | 0 | 0 | 0 | 0 | 0 | 100 |
| Bsp. 2 + MAD | 7,5 + 3 | 0 | 0/3 | 99 | 0 | 0 | 0 | 0 | 0 | 0 | 100 |
| Bsp.2 + MAD | 10 + 3 | 0 | 0/3 | 85 | 0 | 0 | 0 | 0 | 0 | 0 | 91 |

## Patentansprüche

**1.** Benzimidazole der Formel (I)

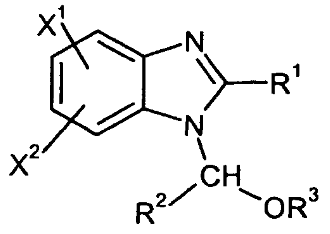

(I),

in welcher

$R^1$ für Fluoralkyl steht,

$R^2$ für Wasserstoff oder Alkyl stehl

R3 für Alkyl steht,

$X^1$ für Trifluormethyl steht

$X^2$ für Trifluormethoxy steht,

oder deren Salze.

**2.** Verfahren zur Herstellung von Verbindungen gemäß Anspruch 1 der Formel (I)

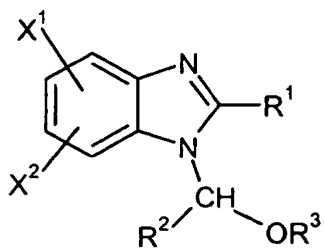

(I),

in welcher
$R^1$, $R^2$, $R^3$, $X^1$ und $X^2$ die in Anspruch 1 genannten Bedeutungen haben,
bei dem man
1 H-Benzimidazole der Formel (II)

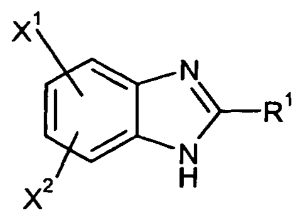

(II),

in welcher
$R^1$, $X^1$ und $X^2$ die in Anspruch 1 angegebene Bedeutung haben,
mit einem Alkylierungsmittel der Formel (III)

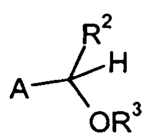

(III),

in welcher

A für eine geeignete Abgangsgruppe steht,

$R^2$ und $R^3$ die in Anspruch 1 angegebene Bedeutung haben,

gegebenenfalls in Gegenwart von Verdünnungsmitteln und/oder Reaktionshilfsmitteln umsetzt.

**3.** Mittel, **gekennzeichnet durch** einen Gehalt an mindestens einer Verbindung der Formel (I) gemäß Anspruch 1.

**4.** Mittel gemäß Anspruch 3, **dadurch gekennzeichnet, dass** es als weiteren Wirkstoff ein Polyetherantibiotikum oder ein synthetisch hergestelltes Coccidiosemittel enthält.

5. Mittel gemäß Anspruch 4, **dadurch gekennzeichnet**, das Polyetherantibiotikum oder das synthetisch hergestellte Coccidiosemittel ausgewählt ist aus der Gruppe bestehend aus: Amprolium, Robenidin, Toltrazuril, Monensin, Salinomycin, Maduramicin, Lasalocid, Narasin, Semduramicin.

6. Verfahren zur Herstellung von Antiparasitika, **dadurch gekennzeichnet, dass** man Verbindungen der Formel (I) gemäß Anspruch 1 mit Streckmitteln und/oder oberflächenaktiven Mitteln vermischt.

7. Verwendung von Verbindungen der Formel (I) gemäß Anspruch 1 zur Herstellung von Antiparasitika.

8. Verwendung gemäß Anspruch 7 zur Herstellung von Mitteln zur Bekämpfung von Coccidien.

## Claims

1. Benzimidazoles of the formula (I)

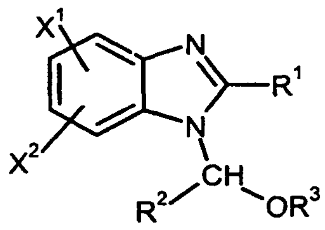

(I),

in which

$R^1$ represents fluoroalkyl,

$R^2$ represents hydrogen or alkyl,

$R^3$ represents alkyl,

$X^1$ represents trifluoromethyl,

$X^2$ represents trifluoromethoxy

or salts thereof.

2. Process for preparing compounds according to Claim 1 of the formula (I)

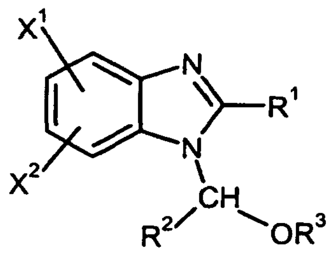

(I),

in which
$R^1$, $R^2$, $R^3$, $X^1$ and $X^2$ have the meanings given in Claim 1,
**characterized in that**
1 H-benzimidazoles of the formula (II)

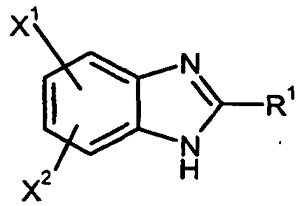

(II),

in which
$R^1$, $X^1$ and $X^2$ have the meaning given in Claim 1,
are reacted with an alkylating agent of the formula (III)

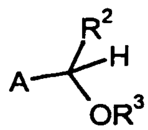

(III),

in which

A represents a suitable leaving group,

$R^2$ and 3 have the meaning given in Claim 1,

if appropriate in the presence of diluents and/or reaction auxiliaries.

3. Compositions, **characterized in that** they comprise at least one compound of the formula (I) according to Claim 1.

4. Compositions according to Claim 3, **characterized in that** they comprise, as further active compound, a polyether antibiotic or a synthetic coccidiosis agent.

5. Compositions according to Claim 4, **characterized in that** the polyether antibiotic or the synthetic coccidiosis agent is selected from the group consisting of: amprolium, robenidin, toltrazuril, monensin, salinomycin, maduramicin, lasalocid, narasin, semduramicin.

6. Process for preparing antiparasitic compositions, **characterized in that** compounds of the formula (I) according to Claim 1 are mixed with extenders and/or surfactants.

7. Use of compounds of the formula (I) according to Claim 1 for preparing antiparasitic compositions.

8. Use according to Claim 7 for preparing compositions for the control of coccidia.

## Revendications

1. Benzimidazoles de formule (I)

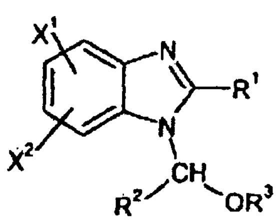

(I),

dans laquelle

$R^1$ est un reste fluoralkyle,
$R^2$ représente l'hydrogène ou un reste alkyle,
$R^3$ est un reste alkyle,
$X^1$ est un reste trifluorométhyle,
$X^2$ est un reste trifluorométhoxy,

ou leurs sels.

**2.** Procédé de production de composés suivant la revendication 1, de formule (I)

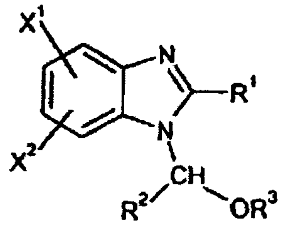

(I),

dans laquelle
$R^1$, $R^2$, $R^3$, $X^1$ et $X^2$ ont les définitions mentionnées dans la revendication 1,
procédé dans lequel
on fait réagir des 1-H-benzimidazole de formule (II)

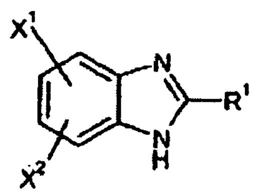

(II),

dans laquelle
$R^1$, $X^1$ et $X^2$ ont la définition indiquée ci-dessus, avec un agent d'alkylation de formule (III)

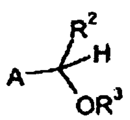

(III),

dans laquelle
A est un groupe partant convenable,
$R^2$ et $R^3$ ont la définition indiquée dans la revendication 1, éventuellement en présence de diluants et/ou d'auxiliaires de réaction.

**3.** Composition **caractérisée par** une teneur en au moins un composé de formule (I) suivant la revendication 1.

**4.** Composition suivant la revendication 3, **caractérisée en ce qu'**elle contient comme autre substance active un antibiotique de type polyéther ou un agent contre la coccidiose préparé par synthèse.

**5.** Composition suivant la revendication 4, **caractérisée en ce que** l'antibiotique du type polyéther ou l'agent contre la coccidiose préparé par synthèse est choisi dans le groupe constitué de : amprolium, robénidine, toltrazuril, monensine, salinomycine, maduramicine, lasalocide, narasine, semduramicine.

6. Procédé de préparation de compositions antiparasitaires, **caractérisé en ce qu'**on mélange des composés de formule (I) suivant la revendication 1 avec des diluants et/ou des agents tensioactifs.

7. Utilisation de composés de formule (I) suivant la revendication 1, pour la préparation de compositions antiparasitaires.

8. Utilisation suivant la revendication 7, pour la préparation de compositions destinées à combattre des coccidies.